# EUROPEAN PATENT APPLICATION

(11) **EP 1 727 397 A1**
(43) Date of publication of application: **29.11.2006**
(21) Application number: 05720688.0
(22) Date of filing: 14.03.2005
(51) Int. Cl.: H05B 33/14, C09K 11/06

(54) **MATERIAL FOR ORGANIC ELECTROLUMINESCENT ELEMENT AND ORGANIC ELECTROLUMINESCENT ELEMENT EMPLOYING THE SAME**

(30) Priority: 17.03.2004 JP 2004076047
(71) Applicant: IDEMITSU KOSAN CO., LTD., Tokyo 100-8321 (JP)
(72) Inventor: ARAKANE, Takashi, 2990293 (JP); IWAKUMA, Toshihiro, 2990293 (JP); IKEDA, Hidetsugu, 2990293 (JP); NAKAMURA, Hiroaki, 2990293 (JP); WATANABE, Ryusuke, 2990293 (JP); IKEDA, Kiyoshi, 2990293 (JP)
(74) Representative: Gille Hrabal Struck Neidlein Prop Roos
(86) International application number: PCT/JP2005/004417
(87) International publication number: WO 2005/089027

(57) **Abstract**

A material for an organic electroluminescence device includes a compound composed of a specific structure showing enhanced steric hindrance. An organic electroluminescence device includes an organic thin film layer composed of one or more layers including at least a light emitting layer, the organic thin film layer is sandwiched between a cathode and an anode, in which at least one layer of the organic thin film layer contains the material for organic electroluminescence device. There are provided the organic electroluminescence device and the material for an organic electroluminescence device having a high luminescent efficiency, excellent heat resistance, and a long lifetime.

## Description

### TECHNICAL FIELD

The present invention relates to a material for an organic electroluminescence device and an organic electroluminescence device using the same. More specifically, the present invention relates to a material for an organic electroluminescence device and an organic electroluminescence device each having a high luminescent efficiency, excellent heat resistance, and a long lifetime.

### BACKGROUND ART

An organic electroluminescence device (hereinafter, the term "electroluminescence" is sometimes abbreviated to "EL") is a spontaneous light emitting device which utilizes the principle that a fluorescent substance emits light by energy of recombination of holes injected from an anode and electrons injected from a cathode when an electric field is applied. Since an organic EL device of the laminate type driven under a low electric voltage was reported by C. W. Tang of Eastman Kodak Company (C. W. Tang and S. A. Vanslyke, Applied Physics Letters, Volume 51, Pages 913, 1987), many studies have been conducted on the organic EL device using organic materials as the constituting materials. Tang et al. used tris(8-hydroxyquinolinol)aluminum for the light emitting layer and a triphenyldiamine derivative for the hole transporting layer. Advantages of the laminate structure include that the efficiency of hole injection into the light emitting layer can be increased, that the efficiency of forming excitons which are formed by blocking and recombining electrons injected from the cathode can be increased, and that excitons formed among the light emitting layer can be enclosed. As the device structure of the organic EL device, a two-layered structure having a hole transporting (injecting) layer and an electron transporting and light emitting layer and a three-layered structure having a hole transporting (injecting) layer, a light emitting layer and an electron transporting (injecting) layer are well known. To increase the efficiency of recombination of injected holes and electrons in the device of the laminate type structure, the structure of the device and the process for forming the device have been studied.
As the light emitting material of the organic EL device, chelate complexes such as tris(8-quinolinolato)aluminum complexes, coumarine derivatives, tetraphenylbutadiene derivatives, distyrylarylene derivatives and oxadiazole derivatives are known. It is reported that light in the visible region ranging from blue light to red light can be obtained by using these light emitting materials, and development of a device exhibiting color images is expected (see, for example, Patent Document 1, Patent Document 2, and Patent Document 3).
It is recently proposed that a phosphorescent materials is used in the light emitting layer of an organic EL device other than a fluorescent material (see, for example, Non-Patent Document 1 and Non-Patent Document 2). As described above, a great efficiency of light emission is achieved by utilizing an organic phosphorescent material excited to the singlet state and the triplet state in the light emitting layer of an organic EL device. It is considered that singlet excimers and triplet excimers are formed in relative amounts of 1:3 due to the difference in the multiplicity of spin when electrons and holes are recombined in an organic EL device. Therefore, it is expected that an efficiency of light emission 3 to 4 times as great as that of a device utilizing fluorescence alone can be achieved by utilizing a phosphorescent light emitting material.

Such organic EL device has used a constitution in which layers such as an anode, a hole transporting layer, an organic light emitting layer, an electron transporting layer (hole blocking layer), an electron transporting layer, and a cathode are sequentially laminated in such a manner that an excited state of a triplet or an exciton of the triplet does not quench. Each of a host compound and a phosphorescent compound has been used in the organic light emitting layer (see, for example, Patent Document 4 and Patent Document 5). In those patent documents, 4,4-N,N-dicarbazole biphenyl has been used as a host compound. However, the compound has a glass transition temperature of 110°C or lower. Furthermore, the compound has so good symmetry that the compound is apt to crystallize. In addition, the compound has a problem in that a short circuit or a pixel defect occurs when a device is subjected to a heat test.
It has been also found that, at the time of the vapor deposition of the compound, crystal growth occurs at, for example, a site where foreign matter or a projection of an electrode is present, so a larger number of defects than that in an initial state before a heat resistance test are generated, and the number of defects increases with time. In addition, carbazole derivatives each holding three-fold symmetry have been also used as hosts. However, those derivatives each have good symmetry, so the following phenomenon is inevitable: at the time of vapor deposition, a crystal grows at, for example, a site where foreign matter or an electrode projection is present, so a larger number of defects than that in an initial state before a heat resistance test are generated, and the number of defects increases with time.
Furthermore, patents in each of which a host compound and a phosphorescent compound have been used in an organic light emitting layer have been disclosed (see, for example, Patent Document 6, Patent Document 7, Patent Document 8, Patent Document 9, and Patent Document 10). In Patent Document 6, heat resistance is improved. However, in a phenylene structure constituting a compound, major parts of the structure each adopt a bonding manner in which each of the parts bonds at a para position, and only the central benzene ring of the structure bonds at a meta position, so symmetry is still good and a problem of crystallization is inevitable. In addition, each of Patent Document 7 and Patent Document 8 discloses a host material into which a heterocyclic skeleton such as a triazine skeleton has been introduced in addition to a carbazole skeleton. However, a triazine ring bonds at a para position via phenylene from the carbazole skeleton. As a result, the linearity of a compound is high, so the energy of a host in a triplet excited state reduces, and energy is hardly transmitted from the host to a phosphorescent dopant. In particular, there arises a problem in that a reduction in luminescent efficiency occurs in a blue phosphorescent device. Furthermore, in each of Patent Document 9 and Patent Document 10, a lifetime and heat resistance are improved by adopting a structure in which a diarylaminophenyl group and an aryl group bond to a benzene ring at meta positions in a phenylene structure constituting a compound, but the lifetime and the heat resistance are still insufficient for practical use.

Patent Document 1: JP-A-08-239655
Patent Document 2: JP-A-07-138561
Patent Document 3: JP-A-03-200889
Patent Document 4: US 6,097,147
Patent Document 5: WO01/41512
Patent Document 6: JP-A-2003-31371
Patent Document 7: JP-A-2002-193952
Patent Document 8: EP 1202608
Patent Document 9: JP-A-10-237438
Patent Document 10: WO01/72927
Non Patent Document 1: D. F.O' Brien and M.A.Baldo et al. "Improved energy transfer in electrophosphorescent devices" Applied Physics letters Vol.74 No.3, pp442-444, January 18, 1999
Non Patent Document 2: M.A.Baldo et al. "Very high-efficiency green organic light-emitting devices based on electrophosphorescence" Applied Physics letters Vol. 75 No. 1, pp4-6, July 5, 1999

### DISCLOSURE OF THE INVENTION

### PROBLEMS TO BE SOLVED BY THE INVENTION

The present invention has been made with a view to solving the above problems, and it is an object of the present invention to provide a material for an organic EL device having a high luminescent efficiency, excellent heat resistance, and a long lifetime, and an organic EL device employing the same.

### MEANS FOR SOLVING THE PROBLEMS

The inventors of the present invention have made extensive studies with a view to achieving the above object. As a result, they have found that the use of a compound represented by the following general formula (1), the compound having a large molecular weight and showing enhanced steric hindrance, as a host material can provide an organic EL device having a high efficiency, high heat resistance, and a long lifetime, thereby solving the present invention.

According to the present invention, there is provided a material for an organic electroluminescence device comprising a compound represented by the following general formula (1):

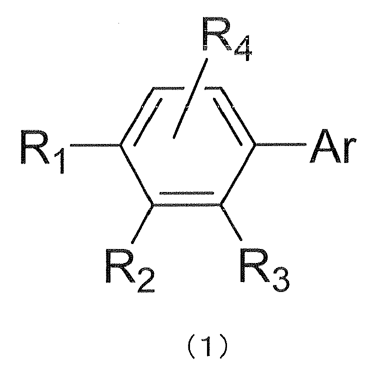

where: Ar represents a group selected from an aryl group which has 6 to 24 ring carbon atoms and which may have a substituent, a carbazolyl group which may have a substituent, and a carbazolylphenyl group which may have a substituent;
R₁ represents a group represented by the following general formula (2) or (3);
at least one of R₂ and R₃ represents a group represented by the following general formula (2) or (3), and the other represents a group represented by the following general formula (2), a group represented by the following general formula (3), a hydrogen atom, or an aryl group which has 6 to 24 ring carbon atoms and which may have a substituent; and
R₄ represents a hydrogen atom or an aryl group which has 6 to 24 ring carbon atoms and which may have a substituent:

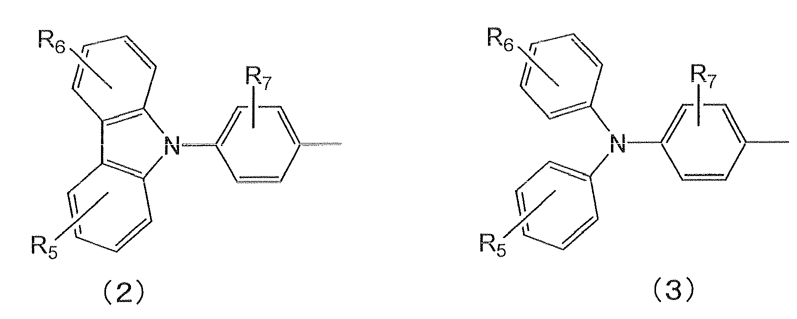

where R₅, R₆, and R₇ each independently represent a hydrogen atom or a substituent.

There is provided the organic EL device comprising an organic thin film layer composed of one or more layers including at least a light emitting layer, the organic thin film layer being sandwiched between a cathode and an anode, wherein at least one layer of the organic thin film layer contains the material for the organic EL device described above.

### EFFECT OF THE INVENTION

The organic EL device employing the material for an organic EL device of the present invention has a high luminescent efficiency, excellent heat resistance, and a long lifetime.

### BEST MODE FOR CARRYING OUT THE INVENTION

A material for an organic EL device of the present invention is composed of a compound represented by the following general formula (1).

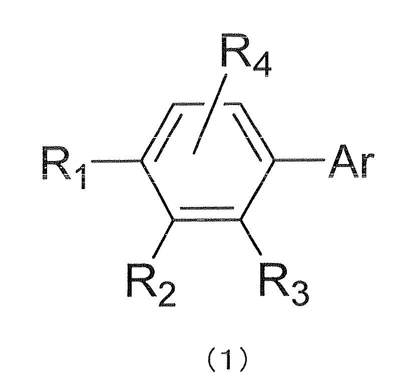

In the general formula (1), Ar represents a group selected from an aryl group which has 6 to 24 (preferably 6 to 18) ring carbon atoms and which may have a substituent, a carbazolyl group which may have a substituent, and a carbazolylphenyl group which may have a substituent.

Examples of the aryl group which has 6 to 24 ring carbon atoms and which may have a substituent include a phenyl group, a 1-naphthyl group, a 2-naphthyl group, a 1-anthryl group, a 2-anthryl group, a 9-anthryl group, a 1-phenanthryl group, a 2-phenanthryl group, a 3-phenanthryl group, a 4-phenanthryl group, a 9-phenanthryl group, a 1-naphthacenyl group, a 2-naphthacenyl group, a 9-naphthacenyl group, a 1-pyrenyl group, a 2-pyrenyl group, a 4-pyrenyl group, a 2-biphenylyl group, a 3-biphenylyl group, a 4-biphenylyl group, a 2,3-diphenylphenyl group, a 2,4-diphenylphenyl group, a 2,5-diphenylphenyl group, a 2,6-diphenylphenyl group, a 3,4-diphenylphenyl group, a 3,5-diphenylphenyl group, a p-terphenyl-4-yl group, a p-terphenyl-3-yl group, a p-terphenyl-2-yl group, an m-terphenyl-4-yl group, an m-terphenyl-3-yl group, an m-terphenyl-2-yl group, an o-tolyl group, an m-tolyl group, a p-tolyl group, a p-t-butylphenyl group, a p-(2-phenylpropyl)phenyl group, a 3-methyl-2-naphthyl group, a 4-methyl-1-naphthyl group, a 4-methyl-1-anthryl group, a 4'-methylbiphenylyl group, a 4"-t-butyl-p-terphenyl-4-ylgroup, an o-cumenyl group, an m-cumenyl group, a p-cumenyl group, a 2,3-xylyl group, a 3,4-xylyl group, a 2,5-xylyl group, a mesityl group, a 1-carbazolyl group, a 2-carbazolyl group, a 3-carbazolyl group, a 4-carbazolyl group, a 9-carbazolyl group, and a 4-(9-carbazolyl)phenyl group.
Of those, a phenyl group, a 2-biphenylyl group, a 3-biphenylyl group, a 4-biphenylyl group, a 2,3-diphenylphenyl group, a 2,4-diphenylphenyl group, a 2,5-diphenylphenyl group, a 2,6-diphenylphenyl group, a 3,4-diphenylphenyl group, a 3,5-diphenylphenyl group, a 1-naphthyl group, a 2-naphthyl group, a 1-phenanthryl group, a 2-phenanthryl group, a 3-phenanthryl group, a 4-phenanthryl group, a 9-phenanthryl group, a 9-carbazolyl group, and a 4-(9-carbazolyl)phenyl group are particularly preferable.

Examples of the substituent for an aryl group, a carbazolyl group, or a carbazolylphenyl group include a halogen atom, a hydroxyl group, an amino group, a nitro group, a cyano group, an alkyl group, an alkenyl group, a cycloalkyl group, an alkoxy group, an aryl group, a heteroaryl group, an aralkyl group, an aryloxy group, and an alkoxycarbonyl group. A linear, branched, or cyclic alkyl or alkoxy group having 1 to 20 carbon atoms and an aryl or heteroaryl group having 1 to 20 carbon atoms are preferable, and an alkyl group having 1 to 8 carbon atoms is more preferable. Examples thereof include a methyl group, an ethyl group, an iso-propyl group, a tert-butyl group, an n-octyl group, an n-decyl group, an n-hexadecyl group, a cyclopropyl group, a cyclopentyl group, and a cyclohexyl group.

In the general formula (1), R₁ represents a group represented by the following general formula (2) or (3).
In the general formula (1), at least one of R₂ and R₃ represents a group represented by the following general formula (2) or (3), and the other represents a group represented by the following general formula (2), a group represented by the following general formula (3), a hydrogen atom, or an aryl group which has 6 to 24 ring carbon atoms and which may have a substituent.

Examples of the aryl group which has 6 to 24 ring carbon atoms and which may have a substituent represented by any one of R₂ and R₃ include examples similar to the above examples for Ar, and the same holds true for preferable examples. Examples of the substituent also include similar examples.
In the general formula (1), R₄ represents a hydrogen atom or an aryl group which has 6 to 24 ring carbon atoms and which may have a substituent.
Examples of the aryl group which has 6 to 24 ring carbon atoms and which may have a substituent represented by R₄ include examples similar to the above examples for Ar, and the same holds true for preferable examples. Examples of the substituent also include similar examples.

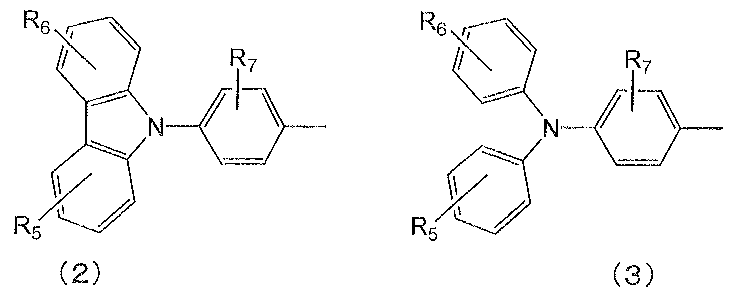

In the general formulae (2) and (3), R₅, R₆, and R₇ each independently represent a hydrogen atom or a substituent.
Examples of the substituent include examples similar to the above examples for Ar, and the same holds true for preferable examples.

In the material for an organic EL device of the present invention, it is preferable that one of R₂ and R₃ in the general formula (1) represents a group represented by the general formula (2) or (3), and the other represents a group represented by the general formula (2), a group represented by the general formula (3), a hydrogen atom, or an aryl group which has 6 to 24 ring carbon atoms and which may have a substituent.
In addition, R₂ or R₃, and R₁ each preferably represent a group represented by the general formula (2).
In addition, the material for an organic EL device of the present invention is preferably a host material in a light emitting layer of an organic EL device.

Specific examples of the material for an organic EL device composed of the compound represented by the general formula (1) of the present invention are shown below. However, the examples are not limited to these exemplified compounds.

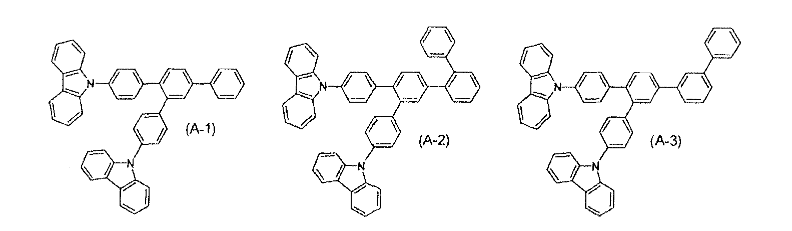

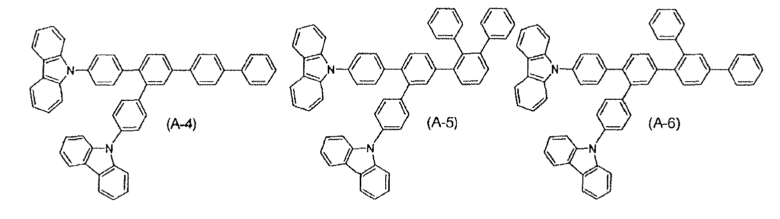

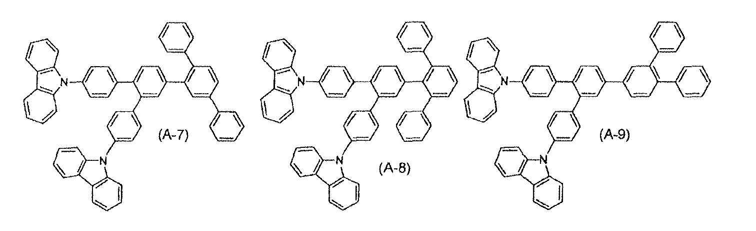

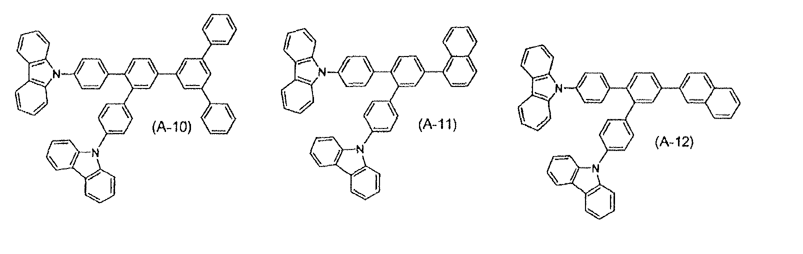

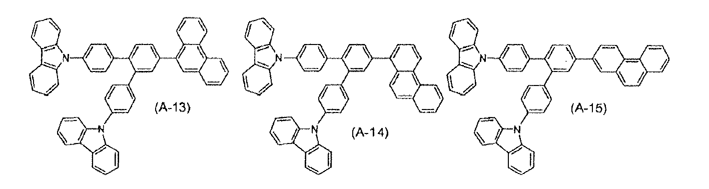

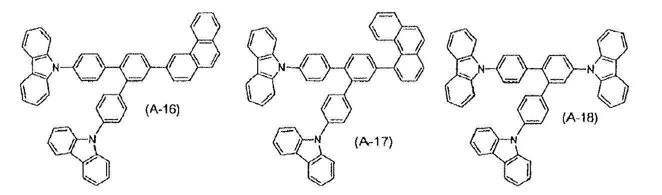

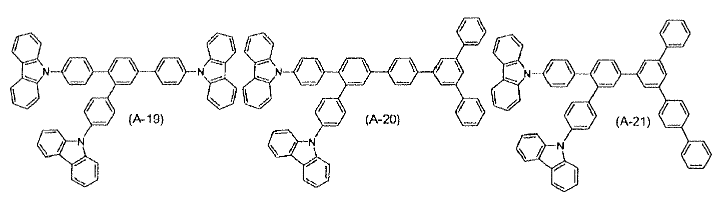

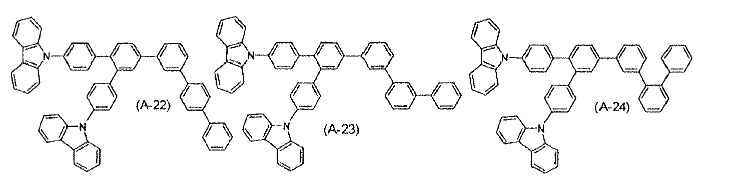

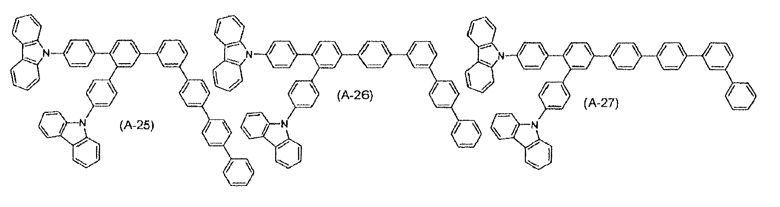

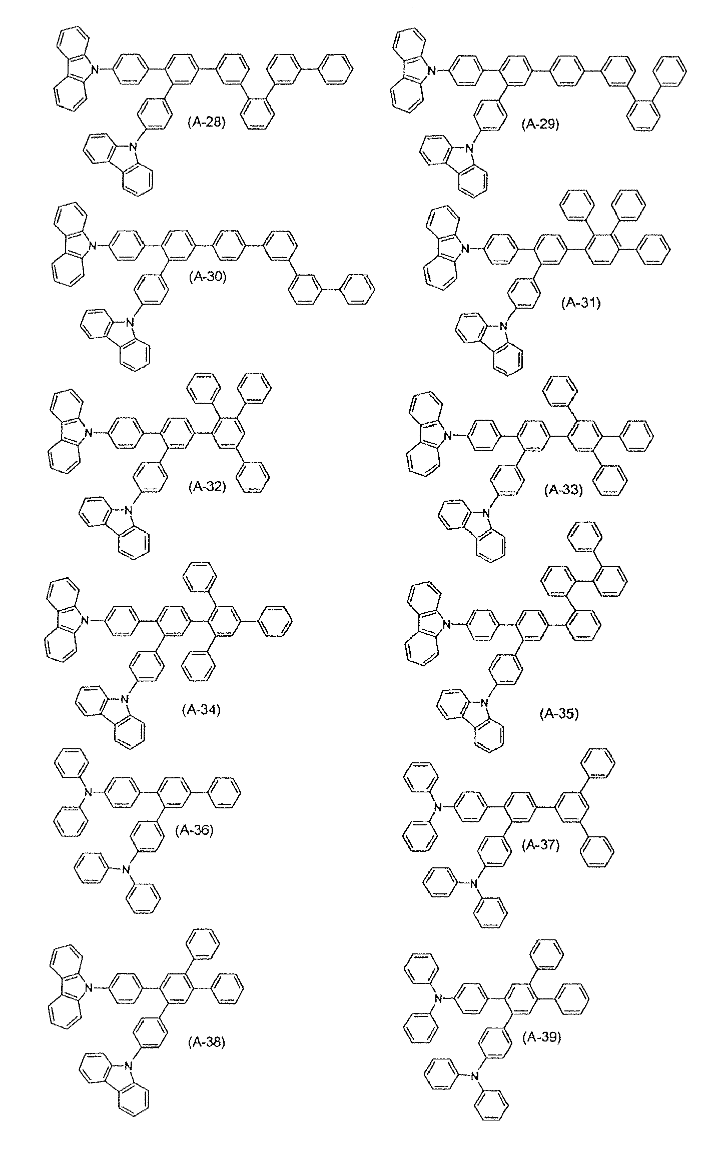

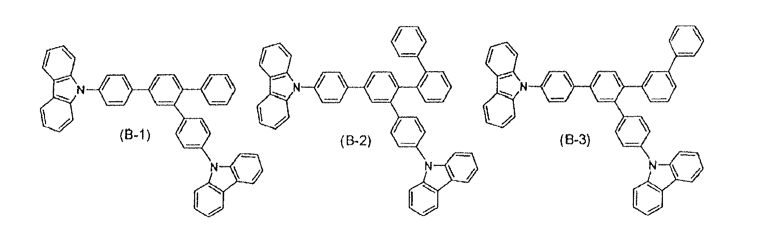

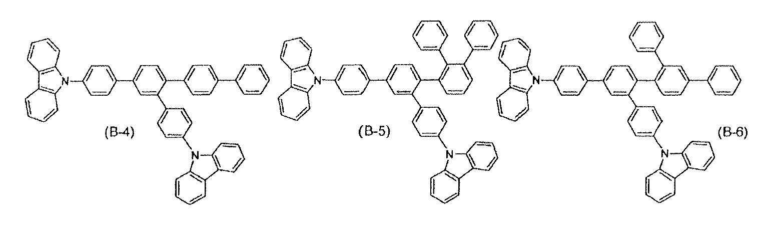

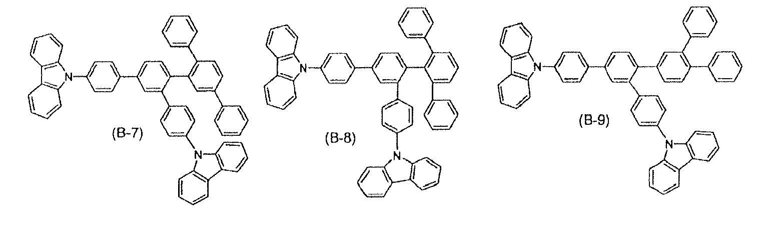

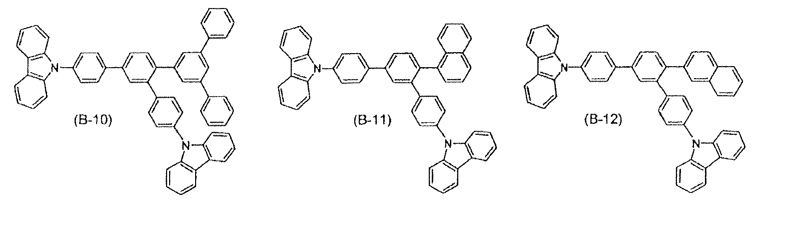

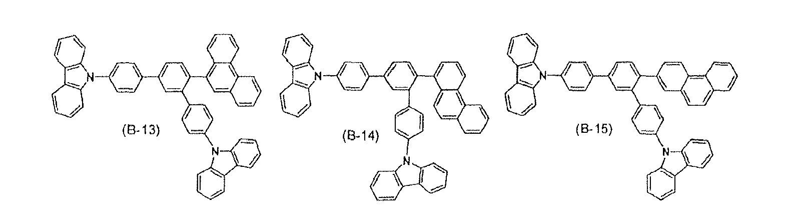

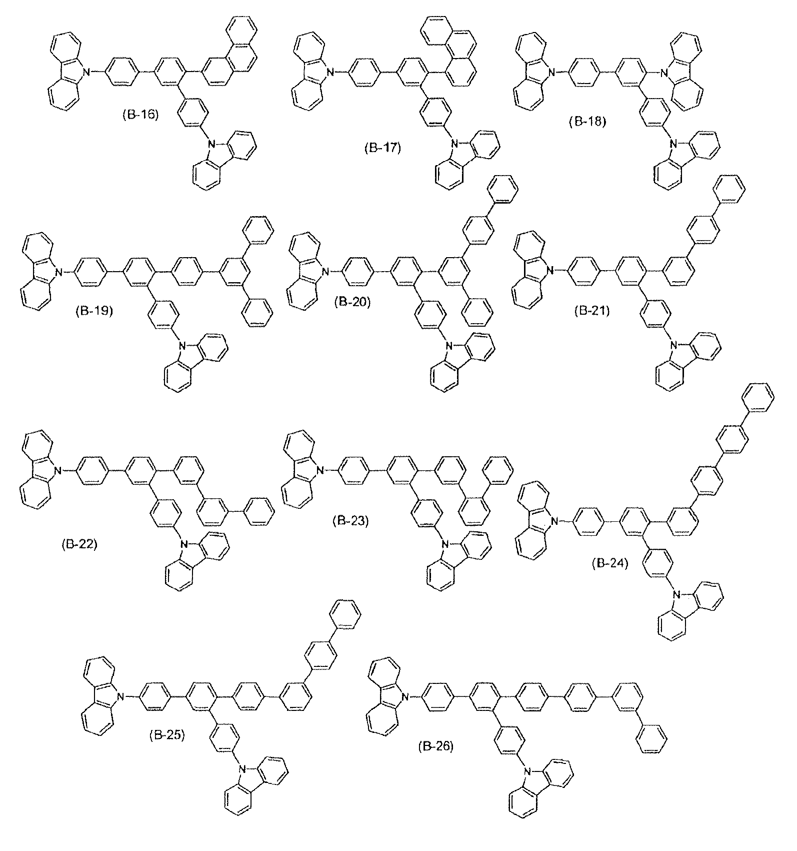

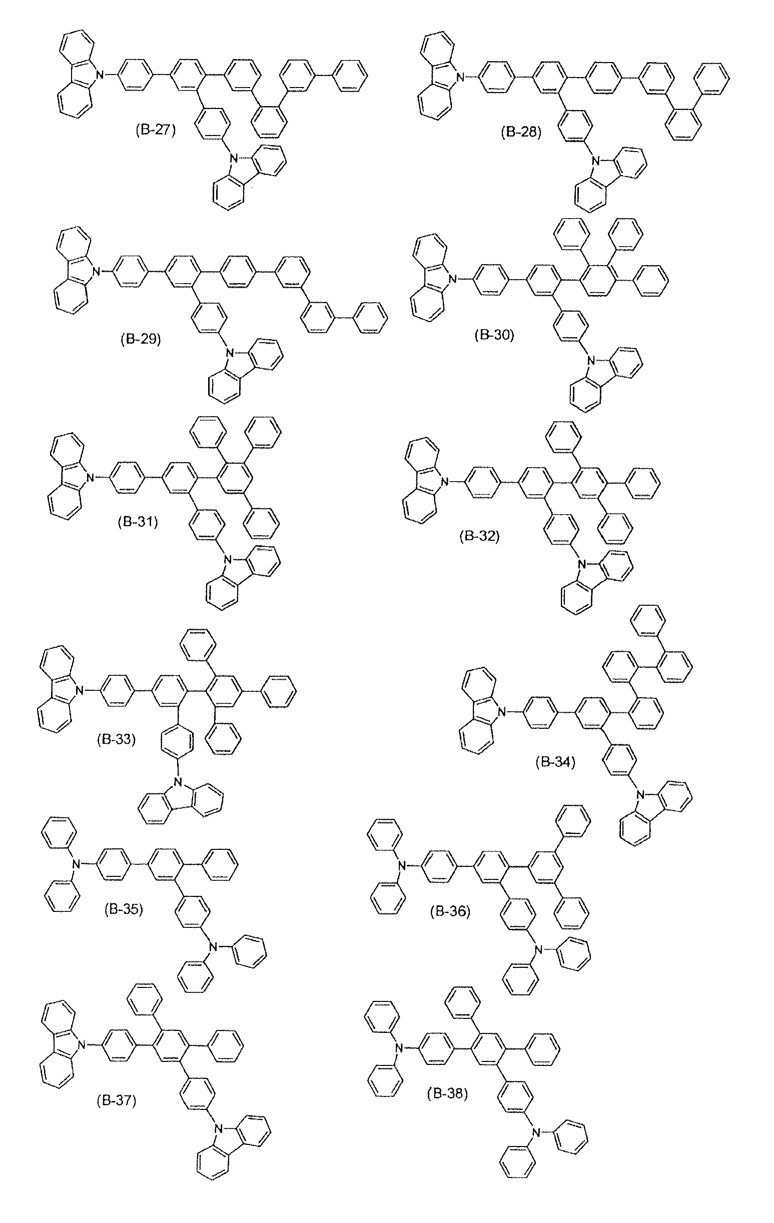

Next, an organic EL device of the present invention will be described.
The organic EL device of the present invention includes an organic thin film layer composed of one or more layers including at least a light emitting layer, the organic thin film layer being sandwiched between a cathode and an anode. In the organic EL device, at least one layer of the organic thin film layer contains the material for an organic EL device of the present invention.
When the organic EL device of the present invention is of a single-layer type, the light emitting layer is provided for a gap between the anode and the cathode. The light emitting layer contains a light emittingmaterial. In addition to the light emitting material, the light emitting layer may contain a hole injecting material or an electron injecting material for transporting a hole injected from the anode or an electron injected from the cathode to the light emitting material. In addition, the light emitting material preferably brings together an extremely high fluorescent quantum efficiency, a high hole transporting ability, and a high electron transporting ability, and preferably forms a uniform thin film. Examples of the constitution of the multilayer-type organic EL device include laminates each having a multilayer construction such as anode/hole injecting layer (hole transporting layer) /light emitting layer/cathode, anode/light emitting layer/electron injecting layer (electron transporting layer) /cathode, anode/hole injecting layer (hole transporting layer) /light emitting layer/electron injecting layer (electron transporting layer)/cathode, or anode/hole injecting layer (hole transporting layer)/light emitting layer/hole blocking layer/electron injecting layer (electron transporting layer)/cathode.

In the organic EL device of the present invention, it is preferable that the light emitting layer or the hole injecting layer (hole transporting layer) contain the material for an organic EL device of the present invention, and it is more preferable that the light emitting layer contain the material for an organic EL device.
The compound represented by the general formula (1) to be used in the present invention is excellent in hole transporting property and enables the stable injection of a hole. In addition, the compound has a high glass transition point (preferably 120 to 160°C), and hardly interacts with the light emitting material, so non-radiative transition caused by the interaction can be avoided. Accordingly, incorporating the compound into at least one layer of the organic thin film layer of the organic EL device improves the luminance, heat resistance, lifetime, and luminescent efficiency of the organic EL device.
In addition, in the organic EL device of the present invention, it is preferable that: the light emitting layer contain a host material and a phosphorescent material; and the host material be the material for an organic EL device composed of the compound represented by the general formula (1) of the present invention.
The above phosphorescent light emitting material is preferably a metal complex containing at least one metal selected from the group consisting of Ir, Ru, Pd, Pt, Os, and Re in terms of a high phosphorescent quantum yield and the ability to improve the external quantum efficiency of a light emitting device additionally. The reason for this is as follows: as long as the phosphorescent material is any one of those metal complexes, when the compound represented by the general formula (1) is used as a host material, energy can be effectively transferred from a triplet exciton from the compound.

More specific examples of the phosphorescent material include metal complexes such as tris(2-phenylpyridine)iridium, tris(2-phenylpyridine)ruthenium, tris(2-phenylpyridine)-palladium, tris(2-phenylpyridine)platinum, tris(2-phenylpyridine)osmium, tris(2-phenylpyridine)rhenium, platinum octaethylporphyrin, platinum octaphenylporphyrin, palladium octaethylporphyrin, palladium octaphenylporphyrin, and bis(4,6-difluorophenylpyridine)picolinate iridium. A metal complex containing Ir, for example, tris(2-phenylpyridine)iridium represented by the following structure is preferable in order to cause an energy transfer to proceed in a more efficient manner to thereby allow fluorescence emission.

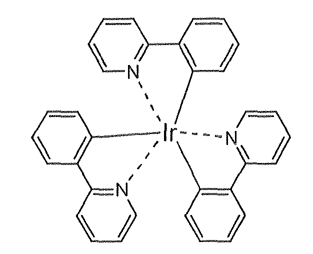

In addition, at least one ligand of the metal complex preferably has a phenylpyridine skeleton, a bipyridyl skeleton, or a phenanthroline skeleton. The reason for this is that the presence of any one of those electron-withdrawing skeletons in a molecule enables energy to be effectively transferred from a triplet of a carbazole derivative. In particular, among those skeletons, a material having a phenylpyridine skeleton such as tris(2-phenylpyridine)iridium is a more preferable phosphorescent material.
In addition, the loading of the phosphorescent material is preferably 0.1 to 30 parts by weight, more preferably 0.5 to 20 parts by weight, or particularly preferably 1 to 15 parts by weight with respect to 100 parts by weight of the compound of the general formula (1). The reason for this is as follows: when the loading of the phosphorescent material is 0.1 part by weight or more, an effect of the addition of the material is exerted, and energy can be effectively transferred from a triplet exciton of the compound of the general formula (1) while when the loading of the phosphorescent material is 30 parts by weight or less, the phosphorescent material can be uniformly blended with ease, and emission luminance does not vary.

In the organic EL device of the present invention, a known hole injecting material in the hole injecting layer (hole transporting layer) is preferably a compound which: has an ability to transport a hole, an effect of the injection of a hole from the anode, and an excellent effect of the injection of a hole into the light emitting layer or the light emitting material; prevents the movement of an exciton produced in the light emitting layer to the electron injecting layer; and is excellent in ability to form a thin film. Specific examples thereof include, but not limited to: a phthalocyanine derivative; a naphthalocyanine derivative; a porphyrin derivative; oxazole, oxadiazole, triazole, imidazole, imidazolone, imidazolethione, pyrazoline, pyrazolone, tetrahydroimidazole, oxazole, oxadiazole, hydrazone, acylhydrazone, polyarylalkane, stilbene, butadiene, benzidine-type triphenylamine, styrylamine-type triphenylamine, diamine-type triphenylamine, and derivatives thereof; and a polymer material such as polyvinylcarbazole, polysilane, and a conductive polymer.

Of the knownhole injecting materials as described above, a more efficient hole injecting material is an aromatic tertiary amine derivative or a phthalocyanine derivative. Specific examples of the aromatic tertiary amine derivative include, but not limited to, triphenylamine, tritolylamine, tolyldiphenylamine, N,N'-diphenyl-N,N'-(3-methylphenyl)-1,1'-biphenyl-4,4'-diamine, N,N, N',N'-(4-methylphenyl)-1,1'-phenyl-4,4'-diamine, N,N,N',N'-(4-methylphenyl)-1,1'-biphenyl-4,4'-diamine, N,N'-diphenyl-N,N'-dinaphthyl-1,1'-biphenyl-4,4'-diamine, N,N'-(methylphenyl)-N,N'-(4-n-butylphenyl)-phenanthrene-9,10-diamine, N,N'-di-(naph-thyl-1-yl)-N,N'-diphenyl-4,4'-benzidine, N,N,N',N'-tetrakis(diphenyl-4-yl)-4,4'-benzidine, and N,N-bis(4-di-4-tolylaminophenyl)-4-phenyl-cyclohexane, and an oligomer or a polymer each having any of those aromatic tertiary amine skeletons. Specific examples of the phthalocyanine (Pc) derivative include, but not limited to, phthalocyanine derivatives such as H₂Pc, CuPc, CoPc, NiPc, ZnPc, PdPc, FePc, MnPc, ClAlPc, ClGaPc, ClInPc, ClSnPc, Cl₂SiPc, (HO)AlPc, (HO)GaPc, VOPc, TiOPc, MoOPc, and GaPc-O-GaPc, and naphthalocyanine derivatives.

In the organic EL device of the present invention, the known electron injecting material in the electron injecting layer (electron transporting layer) is preferably a compound which: has an ability to transport an electron, an effect of the injection of an electron from the cathode, and an excellent effect of the injection of an electron into the light emitting layer or the light emitting material; prevents the movement of an exciton produced in the light emitting layer to the hole injecting layer; and is excellent in ability to form a thin film. Specific examples of the compound include, but not limited to, fluorenone, anthraquinodimethane, diphenoquinone, thiopyranedioxide, oxazole, oxadiazole, triazole, imidazole, perylenetetracarboxylic acid, fluorenylidenemethane, anthraquinodimethane, and anthrone, and derivatives of them. In addition, charge injecting property can be improved by adding an electron-accepting substance to the hole injecting material and adding an electron-donating substance to the electron injecting material.
Of the known electron injecting materials, a metal complex compound or a nitrogen-containing five-membered ring derivative is an additionally effective electron injecting material.

Examples of the metal complex compound include, but not limited to, 8-hydroxyquinolinatolithium, bis(8-hydroxyquinolinato)zinc, bis(8-hydroxyquinolinato)copper, bis(8-hydroxyquinolinato)manganese, tris(8-hydroxyquinolinato)-aluminum, tris(2-methyl-8-hydroxyquinolinato)aluminum, tris(8-hydroxyquinolinato)gallium, bis(10-hydroxybenzo[h]quinolinato)-beryllium, bis(10-hydroxybenzo[h]quinolinato)zinc, bis(2-methyl-8-quinolinato)chlorogallium, bis(2-methyl-8-quinolinato)-(o-cresolato)gallium, bis(2-methyl-8-quinolinato)(1-naphtholato)aluminum, and bis(2-methyl-8-quinolinato)(2-naphtholato)gallium.

Examples of the preferred nitrogen-containing five-memebered derivative include an oxazole derivative, a thiazole derivative, an oxadiazole derivative, a thiadiazole derivative, and a triazole derivative. Specific examples of the derivative include, but not limited to, 2,5-bis(1-phenyl)-1,3,4-oxazole, dimethyl POPOP, 2,5-bis(1-phenyl)-1,3,4-thiazole, 2,5-bis(1-phenyl)-1,3,4-oxadiazole, 2-(4'-tert-butylphenyl)-5-(4''-biphenyl)-1,3,4-oxadiazole, 2,5-bis(1-naphthyl)-1,3,4-oxadiazole, 1,4-bis[2-(5-phenyloxadiazolyl)]benzene, 1,4-bis[2-(5-phenyloxadiazolyl)-4-tert-butylbenzene], 2-(4'-tert-butyl-phenyl)-5-(4''-biphenyl)-1,3,4-thiadiazole, 2,5-bis(1-naphthyl)-1,3,4-thiadiazole, 1,4-bis[2-(5-phenylthiadiazolyl)]benzene, 2-(4'-tert-butylphenyl)-5-(4''-biphenyl)-1,3,4-triazole, 2,5-bis(1-naphthyl)-1,3,4-triazole, and 2,4-bis[2-(5-phenyltriazolyl)]-benzene.

In the organic EL device of the present invention, an inorganic compound layer may be provided for a gap between the light emitting layer and the electrode for improving charge injecting property. Examples of a material for such inorganic compound layer include alkali metal compounds (such as a fluoride and an oxide) and alkaline earth metal compounds. Specific examples of the material include LiF, Li₂O, RaO, SrO, BaF₂, and SrF₂.
In addition, a hole blocking layer having a thickness of 5 nm to 5 µm is preferably formed between the light emitting layer and the cathode. Providing the hole blocking layer as mentioned above improves the property with which a hole is trapped in an organic light emitting layer, provides high emission luminance, and enables to be driven at a low voltage. Examples of a known hole blocking material include 2,9-dimethyl-4,7-diphenyl-1,10-phenanthroline and 2,9-diethyl-4,7-diphenyl-1,10-phenanthroline. The hole blocking layer preferably further contains an alkali metal such as Li or Cs. Combining an alkali metal and a constituting component of the hole blocking layer in the hole blocking layer as described above can not only significantly reduce the voltage at which the organic EL device is driven but also lengthen the lifetime of the device. When an alkali metal is incorporated, the content of the alkali metal is preferably 0.01 to 30 wt%, more preferably 0.05 to 20 parts by weight, or particularly preferably 0.1 to 15 parts by weight when the total amount of the hole blocking layer is defined as 100 wt%. The reason for this is as follows: when the content of the alkali metal is 0.01 wt% or more, an effect of the addition of the alkali metal is surely exerted while when the content is 30 wt% or less, the dispersibility of the alkali metal does not become nonuniform, and emission luminance does not vary.

An electro-conductive material having a work function larger than 4 eV is suitably used in the anode of the organic EL device of the present invention. Examples of a material to be used include : carbon, aluminum, vanadium, iron, cobalt, nickel, tungsten, silver, gold, platinum, and palladium, and alloys of them; metal oxides such as tin oxide and indium oxide each used in an ITO substrate or an NESA substrate; and organic conductive resins such as polythiophene and polypyrrole. A conductive substance having a work function smaller than 4 eV is suitably used in the cathode. Examples of a substance to be used include, but not limited to, magnesium, calcium, tin, lead, titanium, yttrium, lithium, ruthenium, manganese, and aluminum, and alloys of them. Representative examples of the alloys include, but not limited to, magnesium/silver, magnesium/indium, and lithium/aluminum. The ratio of an alloy is controlled depending on, for example, the temperature of a deposition source, an atmosphere, and the degree of vacuum, and is selected to be an appropriate ratio. Each of the anode and the cathode may be formed of a layer constitution having two or more layers as required.

A reducing dopant is preferably added to an interfacial region between a cathode and an organic thin layer in the organic EL device of the present invention.
Examples of the reducing dopant include at least one kind selected from an alkali metal, an alkali metal complex, an alkali metal compound, an alkaline earth metal, an alkaline earth metal complex, an alkaline earth metal compound, a rare earth metal, a rare earth metal complex, and a rare earth metal compound.

Examples of the alkali metal include Na having a work function of 2. 36 eV, K having a work function of 2.28 eV, Rb having a work function of 2.16 eV, and Cs having a work function of 1.95 eV, and an alkali metal having a work function of 2.9 eV or less is particularly preferable. Of those, K, Rb, and Cs are preferable. Rb and Cs are more preferable. Cs is most preferable.
Examples of the alkaline earth metal include Ca having a work function of 2.9 eV, Sr having a work function of 2.0 to 2.5 eV, and Ba having a work function of 2.52 eV, and an alkaline earth metal having a work function of 2. 9 or less is particularlypreferable.
Examples of the rare earth metal include Sc, Y, Ce, Tb, and Yb, and a rare earth metal having a work function of 2.9 or less is particularly preferable.
Of those metals, a preferable metal has a particularly high reductive ability, so improvement of light emission intensity and long lifetime can be attained by adding a small amount of the metal to an electron injecting region.

Examples of the alkali metal compound include an alkali oxide such as Li₂O, Cs₂O, or K₂O, and an alkali halide such as LiF, NaF, CsF, or KF. Of those, an alkali oxide or an alkali fluoride such as LiF, Li₂O, or NaF is preferable.
Examples of the alkaline earth metal compound include BaO, SrO, CaO, and mixtures thereof such as BaₓSr₁₋ₓO (0<x<1) and BaₓCa₁₋ₓO (0<x<1). Of those, BaO, SrO, and CaO are preferable.
Examples of the rare earth metal compound include YbF₃, ScF₃, ScO₃, Y₂O₃, Ce₂O₃, GdF₃, and TbF₃. Of those, YbF₃, ScF₃, and TbF₃ are preferable.

The alkali metal complex, alkaline earth metal complex, and rare metal complex are not particularly limited as long as they each include as a metal ion at least one of alkali metal ions, alkaline earth metal ions, and rare earth metal ions. Meanwhile, preferable examples of a ligand include, but not limited to, quinolinol, benzoquinolinol, acridinol, phenanthridinol, hydroxyphenyloxazole, hydroxyphenylthiazole, hydroxyaryloxadiazole, hydroxydiarylthiadiazole, hydroxyphenylpyridine, hydroxyphenylbenzoimidazole, hydroxybenzotriazole, hydroxyfluborane, bipyridyl, phenanthroline, phthalocyanine, porphyrin, cyclopentadiene, β-diketones, azomethines, and derivatives thereof.

For the addition form of the reducing dopant, it is preferable that the reducing dopant is formed in a shape of a layer or an island in the interfacial region. A preferable example of the forming method includes a method in which an organic substance which is a light emitting material or an electron injecting material for forming the interfacial region is deposited at the same time as the reducing dopant is deposited by a resistant heating deposition method, thereby dispersing the reducing dopant in the organic substance. The disperse concentration by molar ratio of the organic compound to the reducing dopant is 100:1 to 1:100, and is preferably 5:1 to 1:5.
In the case where the reducing dopant is formed into the shape of a layer, the light emitting material or electron injecting material which serves as an organic layer in the interface is formed into the shape of a layer. After that, the reducing dopant is solely deposited by the resistant heating deposition method to form a layer preferably having a thickness of 0.1 to 15 nm.
In the case where the reducing dopant is formed into the shape of an island, the light emitting material or electron injecting material which serves as an organic layer in the interface is formed into the shape of an island. After that, the reducing dopant is solely deposited by the resistant heating deposition method to form an island preferably having a thickness of 0.05 to 1 nm.

At least one surface of the organic EL device of the present invention is desirably sufficiently transparent in the light emitting wavelength region of the device so that the device can efficiently emit light. In addition, the substrate is also desirably transparent. A transparent electrode is set by using any one of the above conductive materials with a method such as vapor deposition or sputtering so that predetermined transparency is secured. The electrode of a light emitting surface desirably has a light transmittance of 10% or more. The substrate is not limited as long as it has mechanical strength, thermal strength, and transparency. Examples of the substrate include a glass substrate and a transparent resin film. Examples of the transparent resin film include polyethylene, an ethylene-vinyl acetate copolymer, an ethylene-vinyl alcohol copolymer, polypropylene, polystyrene, poly(methyl methacrylate), poly(vinyl chloride), poly(vinyl alcohol), poly(vinyl butyral), nylon, polyether ether ketone, polysulfone, polyethersulfone, a tetrafluoroethylene-perfluoroalkylvinyl ether copolymer, poly(vinyl fluoride), a tetrafluoroethylene-ethylene copolymer, a tetrafluoroethylene-hexafluoropropylene copolymer, polychlorotrifluoroethylene, poly(vinylidene fluoride), polyester, polycarbonate, polyurethane, polyimide, polyether imide, polyimide, and polypropylene.

To improve the stability of the organic EL device of the present invention against, for example, temperature, humidity, or an atmosphere, the surface of the device can be provided with a protective layer, or the entirety of the device can be protected with silicone oil, a resin, or the like. Any one of: dry film forming methods such as vacuum deposition, sputtering, plasma, and ion plating; and wet film forming methods such as spin coating, dipping, and flow-coating is applicable to the formation of each layer of the organic EL device. A film thickness, which is not particularly limited, must be set to an appropriate film thickness. When the film thickness is excessively large, a large applied voltage is needed for obtaining a predetermined optical output, thereby resulting in a poor efficiency. When the film thickness is excessively small, a pin hole or the like is generated, so sufficient emission luminance cannot be obtained even when an electric field is applied. A film thickness in the range of 5 nm to 10 µm is suitable in ordinary cases, and a film thickness in the range of 10 nm to 0.2 µm is more preferable.
In the case of a wet film forming method, a material of which each layer is formed is dissolved or dispersed in an appropriate solvent such as ethanol, chloroform, tetrahydrofuran, or dioxane before a thin film is formed. The solvent may be any one of them. In addition, an appropriate resin or additive may be used in each organic thin film layer for the purpose of, for example, improving film formability or preventing a pin hole of a film. Examples of a resin that can be used include : insulating resins such as polystyrene, polycarbonate, polyallylate, polyester, polyamide, polyurethane, polysulfone, poly (methyl methacrylate), poly (methyl acrylate), and cellulose, and copolymers of the resins; photoconductive resins such as poly-N-vinylcarbazole and polysilane; and conductive resins such as polythiophene and polypyrrole. Examples of the additive include an antioxidant, a UV absorber, and a plasticizer.

### EXAMPLES

Next, the present invention will be described in more detail by way of examples. However, the present invention is not limited to these examples.

### Intermediate Synthesis Example 1 (4-(carbazolyl-9-yl)phenylboric acid: (Intermediate A))

254 g of 4-bromoiodobenzene (manufactured by Tokyo Chemical Industry Co., Ltd.), 100 g of carbazole (manufactured by Tokyo Chemical Industry Co., Ltd.), 1 g of copper iodide (manufactured by Wako Pure Chemical Industries, Ltd.), and 267 g of potassium phosphate (manufactured by Wako Pure Chemical Industries, Ltd.) were suspended into 500 ml of 1,4-dioxane (manufactured by Wako Pure Chemical Industries, Ltd.). 7 ml of trans-1,2-cyclohexanediamine were added to the suspension, and the whole was refluxed under heat under an argon atmosphere for 14 hours.
After the temperature of the reaction solution had been cooled to room temperature, methylene chloride and water were added to the solution to separate the solution into two layers. After that, an organic layer was sequentially washed with a 5% aqueous solution of hydrochloric acid and water, and was then dried with anhydrous sodium sulfate. An organic solvent was distilled off under reduced pressure. After that, 50 ml of ethanol were added to the resultant, and the precipitated crystal was filtered and sequentially washed with ethanol and normal hexane. Thus, 178 g of bromophenylcarbazole derivative were obtained.
160 g of the derivative were dissolved in 1, 000 ml of toluene (dehydrated solvent manufactured by Wako Pure Chemical Industries, Ltd.) and 1,000 ml of ether (dehydrated solvent manufactured by Wako Pure Chemical Industries, Ltd.). 420 ml of a solution of normal-butyllithium in hexane (manufactured by Wako Pure Chemical Industries, Ltd., 1.6 M) were added to the solution under an argon atmosphere at -40 °C, and the whole was stirred at -40 °C to 0 °C for 1 hour. Next, the temperature of the reaction solution was cooled to -70 °C. A solution prepared by diluting 340 ml of triisopropyl borate (manufactured by Tokyo Chemical Industry Co., Ltd.) with 500 ml of ether (dehydrated solvent manufactured by Wako Pure Chemical Industries, Ltd.) was dropped to the reaction solution, and the whole was stirred at -70 °C for 1 hour. After that, the temperature of the resultant was increased to room temperature, and then the resultant was stirred for 6 hours.
After the reaction solution had been left overnight, 350 ml of 5% hydrochloric acid were dropped to the reaction solution, and the whole was stirred at room temperature for 45 minutes. After the reaction solution had been separated into two layers, an organic layer was washed with a saturated brine and dried with anhydrous sodium sulfate. After the organic solvent had been removed by distillation under reduced pressure to about one-fifth, the precipitated crystal was filtered, and was sequentially washed with a mixed solvent of toluene and normal-hexane, and normal-hexane, whereby 80 g of 4-(carbazolyl-9-yl)phenylboric acid (Intermediate A) were obtained.

Intermediate Synthesis Example 2 (Synthesis of 3,4-bis(4-(carbazolyl-9-yl)phenyl)-1-bromobenzene (Intermediate B))
140 ml of 3N hydrochloric acid and 20 g of 4-bromo-2-iodoaniline (manufactured by SIGMA-ALDRICH) were loaded into a 1-1 three-necked flask. A solution prepared by dissolving 5.1 g of sodium nitrite (manufactured by Wako Pure Chemical Industries, Ltd.) in 30 ml of pure water was dropped to the mixture, and then the whole was stirred for 1 hour.
Next, a solution prepared by dissolving 100 g of potassium iodide (manufactured by Wako Pure Chemical Industries, Ltd.) in 300 ml of pure water was dropped to the resultant, and the whole was stirred for 3 hours.
After the completion of the reaction, the resultant was extracted with methylene chloride, added with 1 g of sodium hydrogensulfite for liquid separation, and dried with anhydrous sodium sulfate, and the solvent was removed by distillation under reduced pressure. The residue was purified by means of a silica gel column, whereby 17.1 g of 4-bromo-1,2-diiodobenzene were obtained.
8.2 g of 4-bromo-1,2-diiodobenzene, 12.1 g of Intermediate A, and 0.9 g of tetrakis(triphenylphosphine)palladium(0) (manufactured by Tokyo Chemical Industry Co., Ltd.) were loaded. A mixed solution of 42 ml of a 2-M aqueous solution of sodium carbonate and 80 ml of dimethoxyethane (DME) (manufactured by KANTO KAGAKU) was added to the mixture, and the whole was refluxed under heat at 90°C for 10 hours.
After the completion of the reaction, the resultant was left until its temperature reached room temperature. Then, the resultant was filtered, and the crystal was washed with DME, water, and methanol in the stated order and dried. As a result, 3.4 g of 3,4-bis(4-(carbazolyl-9-yl)phenyl)-1-bromobenzene (Intermediate B) were obtained.

### Intermediate Synthesis Example 3 (Synthesis of 3,5-diphenylphenylboric acid (Intermediate C))

2.2 1 of water and 2.2 1 of concentrated hydrochloric acid were added to 150 g of 2,4,6-tribromoaniline (manufactured by SIGMA-ALDRICH), and the whole was stirred at room temperature for 30 minutes. After the temperature of the resultant had been cooled to 0 °C, a solution of 60 g of sodium nitrite (manufactured by Wako Pure Chemical Industries, Ltd.) in 120 ml of water was dropped to the resultant at 5 °C or lower. After the dropping, the resultant was stirred at 5 °C or lower for 1 hour, and a solution of 150 g of potassium iodide (manufactured by Wako Pure Chemical Industries, Ltd.) in 150 ml of water was dropped to the resultant at 5 °C or lower. After the resultant had been stirred at 5 °C or lower for 1 hour, the precipitated crystal was separated by filtration, washed with water, dried, and purified by means of silica gel column chromatography, whereby 165 g of 2,4,6-tribromoiodobenzene were obtained.
160 g of 2,4,6-tribromoiodobenzene were dissolved in 1.6 1 of dehydrated tetrahydrofuran (THF) (manufactured by Wako Pure Chemical Industries, Ltd.). 635 ml of 2-M phenylmagnesium bromide (manufactured by Wako Pure Chemical Industries, Ltd.) were injected into the solution, and the whole was refluxed for 2 hours. After the temperature of the resultant had been cooled to 10 °C, 800 ml of a 1-M aqueous solution of hydrochloric acid were dropped to the resultant. After having been washed with 500 ml of a saturated brine, an organic layer was dried with anhydrous magnesium sulfate, and the solvent was removed by distillation under reduced pressure. The residue was purified by means of silica gel column chromatography, whereby 60 g of 3,5-diphenylbromobenzene were obtained.
10.0 g (32 mmol) of 3,5-diphenylbromobenzene were dissolved in 50 ml of toluene and 50 ml of ether. 27 ml (42 mmol) of a solution (1.6 M) of normal-butyllithium in hexane were added to the solution under an argon atmosphere at -16 to -42°C, and the whole was stirred at -42°C to 0 °C for 1 hour. Next, the temperature of the reaction solution was cooled to -70 °C. A solution prepared by diluting 22 ml (97 mmol) of triisopropyl borate with 25 ml of ether was dropped to the reaction solution, and the whole was stirred at -70 °C for 1 hour. After that, the temperature of the resultant was increased to room temperature, and then the resultant was stirred for 6 hours. Furthermore, 70 ml of 5% hydrochloric acid were dropped to the reaction solution, and the whole was stirred at room temperature for 45 minutes. After the reaction solution had been separated into two layers, an organic layer was sequentially washed with 3% hydrochloric acid and a saturated brine, and was dried with anhydrous sodium sulfate. After the organic solvent had been removed by distillation under reduced pressure to about one-fifth, 10 ml of normal-hexane were added. The precipitated crystal was filtered, and was sequentially washed with a mixed solvent of toluene and normal-hexane, and normal-hexane, whereby 7.0 g of 3,5-diphenylphenylboric acid (Intermediate C) were obtained (78% yield).

### Synthesis Example 1 Synthesis of (Compound (A-1))

1.0 g of Intermediate B, 0.2 g of phenylboric acid (manufactured by Tokyo Chemical Industry Co., Ltd.), and 0.1 g of tetrakis(triphenylphosphine)palladium(0) (manufactured by Tokyo Chemical Industry Co., Ltd.) were loaded. A mixed solution of 4 ml of a 2-M aqueous solution of sodium carbonate and 7 ml of DME (manufactured by KANTO KAGAKU) was added to the mixture, and the whole was refluxed under heat at 90 °C for 10 hours.
After the completion of the reaction, the resultant was left until its temperature reached room temperature. Then, the resultant was filtered, and the crystal was washed with DME, water, andmethanol in the stated order and dried. Furthermore, the resultant was purified by means of column chromatography, whereby 0.72 g of a white powder was obtained. The field desorption mass spectrum (FD-MS) of the powder was measured. As a result, a peak with m/z = 636 was obtained for C₄₈H₃₂N₂ = 636. Accordingly, the powder was identified as Compound (A-1) (73% yield).

### Synthesis Example 2 Synthesis of (Compound (A-10))

1.0 g of Intermediate B, 0.45 g of Intermediate C, and 0.1 g of tetrakis(triphenylphosphine)palladium(0) (manufactured by Tokyo Chemical Industry Co., Ltd.) were loaded. A mixed solution of 4 ml of a 2-M aqueous solution of sodium carbonate and 7 ml of DME (manufactured by KANTO KAGAKU) was added to the mixture, and the whole was refluxed under heat at 90 °C for 10 hours.
After the completion of the reaction, the resultant was left until its temperature reached room temperature. Then, the resultant was filtered, and the crystal was washed with DME, water, and methanol in the stated order and dried. Furthermore, the resultant was purified by means of column chromatography, whereby 0.82 g of a white powder was obtained. FD-MS of the powder was measured. As a result, a peak with m/z = 789 was obtained for C₆₀H₄₀N₂ = 788. Accordingly, the powder was identified as Compound (A-10) (67% yield).

### Synthesis Example 3 Synthesis of (Compound (A-19))

1.0 g of Intermediate B, 0.48 g of Intermediate A, and 0.1 g of tetrakis(triphenylphosphine)palladium(0) (manufactured by Tokyo Chemical Industry Co., Ltd.) were loaded. A mixed solution of 4 ml of a 2-M aqueous solution of sodium carbonate and 7 ml of DME (manufactured by KANTO KAGAKU) was added to the mixture, and the whole was refluxed under heat at 90 °C for 10 hours.
After the completion of the reaction, the resultant was left until its temperature reached room temperature. Then, the resultant was filtered, and the crystal was washed with DME, water, andmethanol in the stated order and dried. Furthermore, the resultant was purified by means of column chromatography, whereby 0.71 g of a white powder was obtained. FD-MS of the powder was measured. As a result, a peak with m/z = 802 was obtained for C₆₀H₃₉N₃ = 801. Accordingly, the powder was identified as Compound (A-19) (57% yield).

### Synthesis Example 4 Synthesis of (Compound (B-10))

140 ml of 3N hydrochloric acid and 17 g of 2, 4-dibromoaniline (manufactured by SIGMA-ALDRICH) were loaded into a 1-1 three-necked flask. A solution prepared by dissolving 5.1 g of sodium nitrite (manufactured by Wako Pure Chemical Industries, Ltd.) in 30 ml of pure water was dropped to the mixture, and then the whole was stirred for 1 hour.
Next, a solution prepared by dissolving 100 g of potassium iodide (manufactured by Wako Pure Chemical Industries, Ltd.) in 300 ml of pure water was dropped to the resultant, and the whole was stirred for 3 hours.
After the completion of the reaction, the resultant was extracted with methylene chloride, added with 1 g of sodium hydrogensulfite for liquid separation, and dried with anhydrous sodium sulfate, and the solvent was removed by distillation under reduced pressure. The residue was purified by means of a silica gel column, whereby 10.3 g of 2,4-dibromo-1-iodobenzene were obtained.
8.0 g of 2,4-dibromo-1-iodobenzene, 9. 0 g of Intermediate C, and 0.75 g of tetrakis(triphenylphosphine)palladium(0) (manufactured by Tokyo Chemical Industry Co., Ltd.) were loaded. A mixed solution of 35 ml of a 2-M aqueous solution of sodium carbonate and 64 ml of dimethoxyethane (DME) (manufactured by KANTO KAGAKU) was added to the mixture, and the whole was refluxed under heat at 90 °C for 10 hours.
After the completion of the reaction, the resultant was left until its temperature reached room temperature. Then, the resultant was filtered, and the crystal was washed with DME, water, andmethanol in the stated order and dried. As a result, 10.1 g of dibromo compound were obtained.
9.5 g of dibromo compound, 12.1 g of Intermediate A, and 0.9 g of tetrakis(triphenylphosphine)palladium(0) (manufactured by Tokyo Chemical Industry Co., Ltd.) were loaded. A mixed solution of 42 ml of a 2-M aqueous solution of sodium carbonate and 80 ml of DME (manufactured by KANTO KAGAKU) was added to the mixture, and the whole was refluxed under heat at 90 °C for 10 hours.
After the completion of the reaction, the resultant was left until its temperature reached room temperature. Then, the resultant was filtered, and the crystal was washed with DME, water, and methanol in the stated order and dried. Furthermore, the resultant was purified by means of column chromatography, whereby 6. 9 g of a white powder was obtained. FD-MS of the powder was measured. As a result, a peak with m/z = 789 was obtained for C₆₀H₄₀N₂ = 788. Accordingly, the powder was identified as Compound (B-10) (44% yield).

### Example 1 (Production of organic EL device: green light emission)

A glass substrate equipped with an ITO transparent electrode measuring 25 mm wide by 75 mm long by 0.7 mm thick was subjected to ultrasonic cleaning in isopropyl alcohol for 5 minutes. After that, the substrate was subjected to UV ozone cleaning for 30 minutes. The glass substrate equipped with the transparent electrode after the cleaning was mounted on a substrate holder of a vacuum deposition device. First, the following copper phthalocyanine film (hereinafter abbreviated as the "CuPc film") having a thickness of 10 nm was formed on the surface of the side where the transparent electrode was formed in such a manner that the film would cover the transparent electrode. The CuPc film functions as a hole injecting layer. After the CuPC film was formed, the following 4,4'-bis[N-(1-naphthyl)-N-phenylamino]biphenyl film (hereinafter abbreviated as the "α-NPD film") having a thickness of 30 nm was formed on the CuPc film. The α-NPD film functions as a hole transporting layer. Furthermore, after the α-NPD film was formed, Compound (A-1) described above and having a thickness of 30 nm was deposited as a host material from the vapor on the α-NPD film, to form a light emitting layer. At the same time, tris(2-phenylpyridine)iridium (hereinafter abbreviated as "Ir(ppy)₃") was added as the following phosphorescent Ir metal complex dopant. The concentration of Ir(ppy)₃ in the light emitting layer was set to 5 wt%. The film functions as a light emitting layer. On the light emitting layer, the following (1,1'-bisphenyl-4-olato)bis-(2-methyl-8-quinolinolato)aluminum (hereinafter abbreviated as the "BAlq film") was formed into a film having a thickness of 10 nm. The BAlq film functions as a hole blocking layer. Furthermore, the following aluminum complex of 8-hydroxyquinoline (hereinafter abbreviated as the "Alq film") was formed into a film having a thickness of 40 nm on the film. The Alq film functions as an electron injecting layer. After that, LiF as an alkali metal halide was deposited from the vapor to have a thickness of 0.2 nm, and then aluminum was deposited from the vapor to have a thickness of 150 nm. The Al/LiF functions as a cathode. Thus, an organic EL device was produced.
The device was subjected to a current test. As a result, green light having an emission luminance of 99 cd/m² was emitted at a voltage of 5.7 V and a current density of 0.26 mA/cm². Chromaticity coordinates were (0.32, 0.61), and a current efficiency was 38.7 cd/A. In addition, the device was driven at a constant current and an initial luminance of 5, 000 cd/m². The time required for the initial luminance to reduce in half to a luminance of 2,500 cd/m² was 622 hours. Furthermore, a heat resistance test was performed. That is, the device was fed with electric current under an environmental condition of 105°C, and a heat-resistant current light emitting time was measured. As a result, it was confirmed that green light having a sufficient emission luminance was emitted even after the lapse of 500 hours from starting to feed the electric current. Table 1 shows the results.

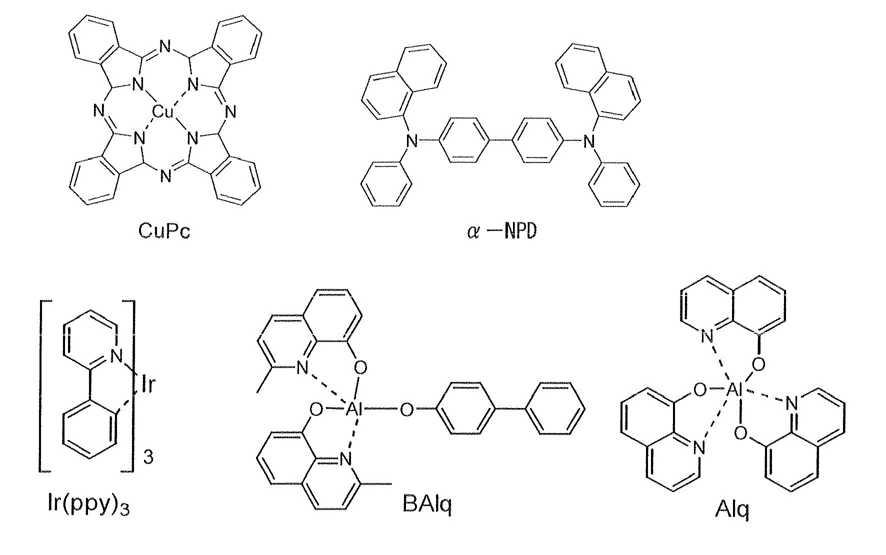

### Examples 2 to 4 (Production of organic EL devices: green light emission)

Organic EL devices were each produced in the same manner as in Example 1 except that a compound described in Table 1 was used instead of Compound (A-1) as a host material for a light emitting layer. A voltage, a current density, a luminance, a current efficiency, a chromaticity, a luminance half life, and a heat-resistant current light emitting time were each measured in the same manner as in Example 1. Table 1 shows the measurements.

### Comparative Examples 1 to 4 (Production of organic EL devices: green light emission)

Organic EL devices were each produced in the same manner as in Example 1 except that the following compound described in Table 1 was used instead of Compound (A-1) as a host material for a light emitting layer. A voltage, a current density, a luminance, a current efficiency, a chromaticity, a luminance half life, and a heat-resistant current light emitting time were each measured in the same manner as in Example 1. Table 1 shows the measurements.

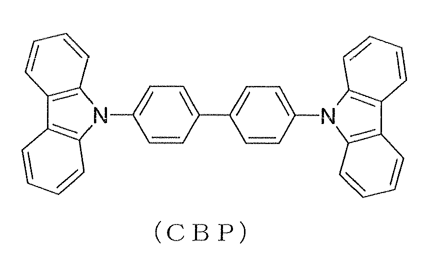

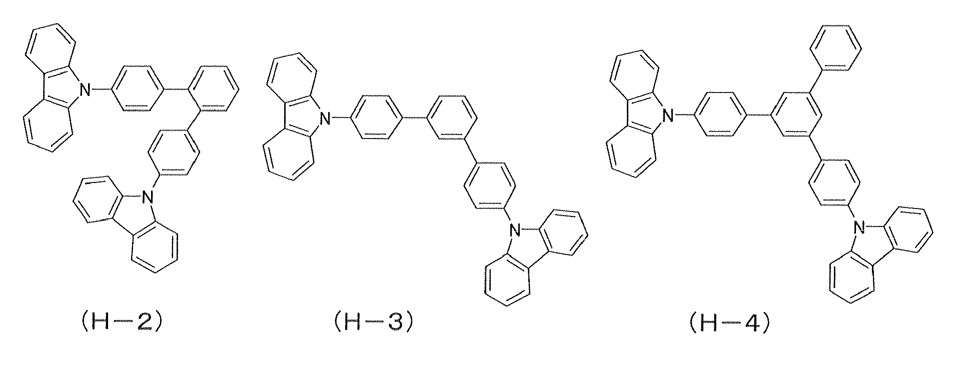

### [Table 1]

**Table 1**

| | Host material for light emitting layer | Voltage (V) | Current density (mA/cm²) | Luminance (cd/m²) | Current efficiency (cd/A) | Chromaticity coordinates (x,y) | Luminance half life (hours) Initial luminance 5,000 (cd/m² ) | Heat-resis tant current light emitting time (hours) |
|---|---|---|---|---|---|---|---|---|
| Example 1 | A-1 | 5.7 | 0.26 | 99 | 38.7 | (0.32,0.61) | 622 | Longer than 500 |
| Example 2 | A-10 | 5.4 | 0.26 | 104 | 39.4 | (0.32,0.61) | 858 | Longer than 500 |
| Example 3 | A-19 | 5.3 | 0.25 | 102 | 40.4 | (0.32,0.61) | 792 | Longer than 500 |
| Example 4 | B-10 | 5.6 | 0.23 | 98 | 43.1 | (0.32,0.61) | 907 | Longer than 500 |
| Comparative Example 1 | CBP | 5.4 | 0.31 | 101 | 32.6 | (0.32,0.61) | 405 | 4 |
| Comparative Example 2 | H-2 | 5.4 | 0.32 | 103 | 32.2 | (0.32,0.61) | 357 | 160 |
| Comparative Example 3 | H-3 | 5.6 | 0.30 | 99 | 33.0 | (0.32,0.61) | 429 | 200 |
| Comparative Example 4 | H-4 | 5.5 | 0.30 | 104 | 35.1 | (0.33,0.61) | 430 | 400 |

As shown in Table 1, an organic EL device employing the compound of the present invention has a high efficiency and a long lifetime, and emits highly heat-resistant green light.

### Example 5 (Production of organic EL device: red light emission)

An organic EL device was produced in the same manner as in Example 1 except that: Compound (B-10) shown above was used instead of Compound (A-1) as a host material for a light emitting layer; and bis(2-benzothieriylpyridine)acetylacetonatoiridium (Ir(btp)₂(acac)) shown below was used instead of Tr(ppy)₃ as a phosphorescent metal complex dopant.
The device was subjected to a current test. As a result, red light having an emission luminance of 100 cd/m² was emitted at a voltage of 7.4 V and a current density of 2.3 mA/cm². Chromaticity coordinates were (0.66, 0.32), and a current efficiency was 4.3 cd/A. In addition, the device was driven at a constant current and an initial luminance of 500 cd/m². The time required for the initial luminance to reduce in half to a luminance of 250 cd/m² was 1742 hours. Furthermore, a heat resistance test was performed. That is, the device was fed with electric current under an environmental condition of 105°C, and a heat-resistant current light emitting time was measured. As a result, it was confirmed that red light having a sufficient emission luminance was emitted even after the lapse of 500 hours from starting to feed the electric current. Table 2 shows the results.

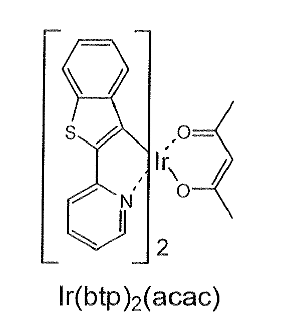

### Example 6 (Production of organic EL devices: red light emission)

An organic EL device was produced in the same manner as in Example 5 except that Compound (A-19) shown above was used instead of Compound (B-10) as a host material for a light emitting layer. A voltage, a current density, a luminance, a current efficiency, a chromaticity, a luminance half life, and a heat-resistant current light emitting time were each measured in the same manner as in Example 5. Table 2 shows the measurements.

### Comparative Example 5 (Production of organic EL devices: red light emission)

An organic EL device was produced in the same manner as in Example 5 except that Compound (CBP) shown above was used instead of Compound (B-10) as a host material for a light emitting layer. A voltage, a current density, a luminance, a current efficiency, a chromaticity, a luminance half life, and a heat-resistant current light emitting time were each measured in the same manner as in Example 5. Table 2 shows the measurements.

### Comparative Examples 6 to 7 (Production of organic EL devices: red light emission)

Organic EL devices were each produced in the same manner as in Example 5 except that Compounds (H-3) and (H-4) shown above and described in Table 2 were, respectively, used instead of Compound (B-10) as a host material for a light emitting layer. A voltage, a current density, a luminance, a current efficiency, a chromaticity, a luminance half life, and a heat-resistant current light emitting time were each measured in the same manner as in Example 5. Table 2 shows the measurements

**Table 2**

| | Host material for light emitting layer | Voltage (V) | Current density (mA/cm²) | Luminance (cd/m²) | Current efficiency (cd/A) | Chromaticity coordinates (x,y) | Luminance half life (hours) Initial luminance 500(cd/m² | Heat-resis tant current light emitting time |
|---|---|---|---|---|---|---|---|---|
| Example 5 | B-10 | 7.4 | 2.3 | 100 | 4.3 | (0.66,0.32) | 1,742 | (hours) Longer than 500 |
| Example 6 | A-19 | 7.0 | 1.7 | 101 | 6.0 | (0.66,0.32) | 1,160 | Longer than 500 |
| Comparative example 5 | CBP | 7.3 | 4.3 | 98 | 2.3 | (0.65,0.33) | 451 | 2 |
| Comparative example 6 | H-3 | 7.3 | 3.5 | 103 | 2.9 | (0.66,0.32) | 769 | 180 |
| Comparative example 7 | H-4 | 7.2 | 3.0 | 100 | 3.3 | (0.66,0.32) | 842 | 340 |

As shown in Table 2, an organic EL device employing the compound of the present invention has a high efficiency and a long lifetime, and emits highly heat-resistant red light.

### INDUSTRIAL APPLICABILITY

As described above in detail, the utilization of the material for an organic electroluminescence device composed of the compound represented by the general formula (1) of the present invention provides an organic electroluminescence device having a high luminescent efficiency, excellent heat resistance, and a long lifetime. Therefore, the organic electroluminescence device of the present invention is extremely useful as, for example, a light source for any one of various electronic instruments.

## Claims

1. A material for an organic electroluminescence device comprising a compound represented by the following general formula (1): 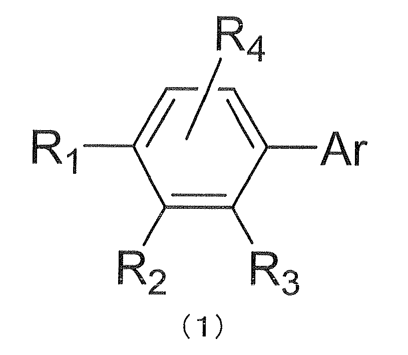 where:
Ar represents a group selected from an aryl group which has 6 to 24 ring carbon atoms and which may have a substituent, a carbazolyl group which may have a substituent, and a carbazolylphenyl group which may have a substituent;
R₁ represents a group represented by the following general formula (2) or (3);
at least one of R₂ and R₃ represents a group represented by the following general formula (2) or (3), and the other represents a group represented by the following general formula (2), a group represented by the following general formula (3), a hydrogen atom, or an aryl group which has 6 to 24 ring carbon atoms and which may have a substituent; and
R₄ represents a hydrogen atom or an aryl group which has 6 to 24 ring carbon atoms and which may have a substituent: 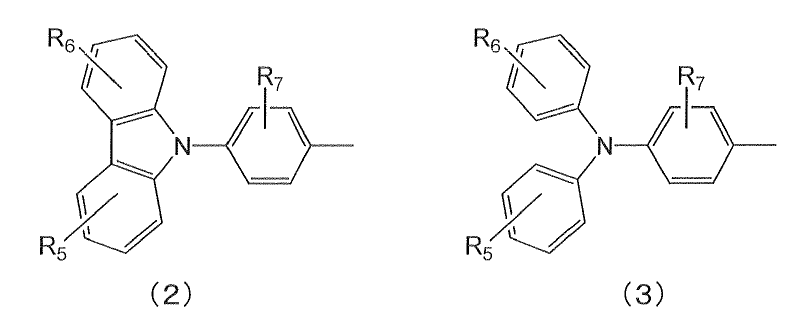
where R₅, R₆, and R₇ each independently represent a hydrogen atom or a substituent.

2. A material for an organic electroluminescence device according to claim 1, wherein one of R₂ and R₃ in the general formula (1) represents a group represented by the general formula (2) or (3), and the other represents a group represented by the general formula (2), a group represented by the general formula (3), a hydrogen atom, or an aryl group which has 6 to 24 ring carbon atoms and which may have a substituent.

3. A material for an organic electroluminescence device according to claim 1, wherein R₂ or R₃, and R₁ each represent a group represented by the general formula (2).

4. A material for an organic electroluminescence device according to claim 1, wherein the material is included as a host material in a light emitting layer of an organic electroluminescence device.

5. An electroluminescence device comprising an anode, a cathode and an organic thin film layer which comprises one layer or a plurality of layers comprising at least a light emitting layer and is disposed between the anode and the cathode, wherein at least one layer in the organic thin film layer comprises the material for an organic electroluminescence device described in Claims 1.

6. An organic electroluminescence device according to claim 5, wherein the light emitting layer contains a host material and a phosphorescent material, and the host material comprises the material for an organic electroluminescence device described in claim 1.

7. An organic electroluminescence device according to claim 5, wherein a reducing dopant is added to an interfacial region between the cathode and the organic thin film layer.
